# FASCICULE DE BREVET EUROPEEN

(11) **EP 1 833 433 B1**
(45) Date de publication et mention de la délivrance du brevet: **20.11.2013**
(21) Numéro de dépôt: 05850673.4
(22) Date de dépôt: 25.11.2005
(51) Int. Cl.: A61F 5/01

(54) **DISPOSITIF DE MAINTIEN EN FLEXION DU PIED D'UNE PERSONNE**
VORRICHTUNG ZUM HALTEN DES FUSSES EINER PERSON IN EINER GEBEUGTEN STELLUNG
DEVICE FOR RETAINING A PERSON'S FOOT IN A FLEXED POSITION

(30) Priorité: 26.11.2004 FR 0412607
(43) Date de publication de la demande: 19.09.2007
(73) Titulaire: Boue, Didier, 36120 Etrechet (FR)
(72) Inventeur: Boue, Didier, 36120 Etrechet (FR)
(74) Mandataire: Thibon-Littaye, Annick
(86) Numéro de dépôt international: PCT/IB2005/003542
(87) Numéro de publication internationale: WO 2006/056868

(56) Documents cités:
- FR-A- 2 586 907
- US-A- 2 584 010
- US-A- 3 986 501
- US-A- 4 441 493
- US-A- 4 817 589
- US-A- 5 860 423

## Description

La présente invention concerne la conception et la réalisation d'un dispositif de maintien en flexion du pied d'une personne, du type comportant des moyens tenseurs pour relever l'avant d'une chaussure portée par le pied vers la jambe. Ce dispositif permet de maintenir le pied dans une position fléchie par rapport à la jambe, c'est-à-dire sensiblement perpendiculaire à l'axe de cette dernière.

Dans toute la présente description, on englobe dans le terme chaussure tout chaussant tel qu'une chaussure proprement dite, un chausson, une pantoufle, une enveloppe de pied en matière synthétique, etc. L'invention s'applique également dans le cas où le pied ne porte pas de chaussure, le dispositif étant alors associé à un accessoire à installer autour du pied.

Le dispositif suivant l'invention est plus particulièrement destiné aux individus souffrant d'une défaillance partielle ou totale des extenseurs et/ou fléchisseurs du pied, qui entraîne le syndrome dit du " pied tombant " ou " pied équin ", ce qui signifie que l'individu ne contrôle plus le mouvement de flexion et d'extension de son pied.

Ainsi, lorsque le pied n'est pas posé à terre, il reste ballant à l'extrémité de la jambe. Les personnes souffrant de ce syndrome doivent, pour marcher, relever très haut la jambe et la lancer exagérément vers l'avant. Pour que ces personnes puissent marcher d'une manière plus naturelle, il est nécessaire que leur pied soit maintenu de façon permanente en position artificiellement fléchie.

Il existe actuellement différents dispositifs pour maintenir le pied d'une personne en position fléchie comme il en est décrit notamment, dans le document FR-2 586 907 dans le document US 2 584 010 et dans le document US 3 986 501.

La présente invention vise à remédier aux inconvénients des dispositifs releveurs de pied existants en proposant un dispositif pour maintenir le pied d'une personne en flexion, qui est confortable à utiliser, facile à mettre en place, et qui confère à son utilisateur une démarche dynamique qui se rapproche au mieux d'une démarche naturelle.

Le dispositif de maintien en flexion du pied d'une personne suivant l'invention est du type de celui du document US 3 986 501. Il comporte des moyens tenseurs qui se montent de manière amovible pour relever l'extrémité avant d'une chaussure portée sur le pied de la personne vers une guêtre se disposant autour de la jambe correspondante. Par extrémité avant, on entend que c'est de préférence toute la partie avant du pied et de la chaussure qui est relevée vers la jambe. Le dispositif suivant l'invention se caractérise en ce que ces moyens tenseurs sont constitués par une sangle élastique qui se dispose en s'étendant symétriquement, à partir d'un point de fixation à l'avant de la chaussure, vers deux points distincts de la guêtre qui sont disposés sur des zones latérales opposées de la jambe, si bien que les efforts de traction exercés par la sangle sont répartis vers ces deux points latéraux, et non sur un point unique à l'avant de la jambe.

On entend par zones latérales de la jambe les deux zones qui s'étendent de part et d'autre du tibia jusqu'à la partie arrière du mollet.

La sangle est suffisamment tendue entre son point de fixation à l'avant de la chaussure et la guêtre pour relever l'avant du pied jusqu'à ce que ce dernier forme un angle qui est de préférence tel que le pied vient se placer en orientation sensiblement perpendiculaire à l'axe de la jambe. Des moyens de blocage sont en outre prévus pour bloquer la sangle dans cette position tendue, si bien que le pied reste en permanence fléchi, quels que soient les mouvements de l'utilisateur. L'invention prévoit avantageusement que la tension de la bande entre le pied et la jambe soit réglable.

Grâce à ces caractéristiques du dispositif selon l'invention, l'effort de traction exercé par la sangle sur la guêtre, afin de relever l'avant du pied vers le tibia, est avantageusement réparti vers deux zones latérales opposées de la guêtre. La traction n'est pas concentrée sur un point unique précis de la jambe, et on évite ainsi avantageusement qu'il se crée une zone douloureuse sur cette dernière. En outre, tant la guêtre que la bande sont avantageusement constituées en des matériaux souples, si bien que la traction s'exerçant d'un élément souple sur un autre élément souple diminue encore les risques d'inconfort ou de blessure pour l'utilisateur.

De plus, le pied est bien maintenu latéralement, si bien qu'on n'observe pas chez les personnes portant ce dispositif de flottement latéral du pied, et que leur démarche paraît plus naturelle. Le pied est d'autant mieux maintenu latéralement que la tension de la sangle entre l'avant du pied et la guêtre est plus forte. Ainsi, une seule opération de tension de la sangle permet à la fois d'amener le pied dans un position perpendiculaire à la jambe, et d'atteindre une position efficace de maintien latéral du pied.

Le caractère naturel de la démarche de l'utilisateur est en outre favorisé par le fait que le dispositif est conçu de telle sorte que la guêtre soit disposée autour de la jambe au-dessus de la cheville, si bien qu'elle n'occasionne pas de gêne au niveau de cette dernière.

Suivant une caractéristique avantageuse de l'invention, la sangle s'accroche par ses deux extrémités sur la face avant de la jambe, en blocage à une longueur assurant un effet tenseur approprié pour relever l'extrémité avant de la chaussure vers la jambe. De ce fait, la tension qu'elle exerce sur la guêtre est non seulement répartie entre deux points latéraux de la jambe, mais également sur toute la face avant de cette dernière. Ceci permet avantageusement de réduire encore les risques que l'utilisateur ressente des douleurs au niveau de la jambe qui seraient dues à la tension exercée sur cette dernière par la sangle.

Suivant une caractéristique avantageuse de l'invention, les deux points de la guêtre vers lesquels s'étend la sangle sont espacés l'un de l'autre de telle sorte que, en liaison avec la position en hauteur de la guêtre sur la jambe, qui est avantageusement choisie de telle sorte que la guêtre soit disposée juste au-dessus de la malléole, la sangle forme avec elle-même, entre le point de fixation à l'avant de ladite chaussure et lesdits deux points respectifs de la guêtre, un angle compris entre 10 et 40 degrés, de préférence environ égal à 25 degrés.

Suivant des modes de réalisation préférés dans la pratique industrielle, l'invention répond aux caractéristiques suivantes, mises en oeuvre séparément ou en chacune de leurs combinaisons techniquement opérantes.

Ces caractéristiques tiennent notamment compte du fait que le dispositif suivant l'invention est destiné à être porté par des personnes, et qu'il doit de ce fait répondre à toutes les exigences de sécurité qu'on peut attendre pour ce type de dispositif. En particulier, le dispositif suivant l'invention est avantageusement conçu de sorte qu'il ne comporte aucun élément qui puisse être susceptible de blesser son utilisateur.

Dans des modes de réalisation préférés de l'invention, le dispositif est entièrement constitué de matériaux légers, si bien qu'il est confortable à porter par l'utilisateur. De plus, ses parties destinées à entrer en contact avec le corps sont souples et flexibles. Le confort d'utilisation de ce dispositif en est encore amélioré.

Suivant une caractéristique avantageuse de l'invention, la sangle, à partir de ses deux points de maintien sur la guêtre, vient se fermer en boucle sur elle-même sur la face avant de la jambe. Cette fermeture est notamment réalisée au moyen d'un système de fixation à position ajustable, et notamment de préférence par des pattes auto-accrochantes, qui permettent des cycles répétés de fermeture et d'ouverture, et qui s'accrochent l'une sur l'autre sur une longueur variable. Ainsi, la sangle peut être fermée sur elle-même en différentes positions, suivant que l'on souhaite relever plus ou moins l'avant du pied vers la jambe, et suivant la morphologie de l'utilisateur, et notamment la taille de sa jambe et de son pied.

Cette caractéristique permet avantageusement de régler en une seule opération le degré de tension de la sangle, en assurant en outre que cette tension soit latéralement équilibrée. Il ne se crée ainsi pas de déséquilibre au niveau du pied.

Dans des modes de réalisation préférés de l'invention, la sangle coulisse librement dans des boucles de maintien montées sur la guêtre, ce qui facilite sa mise en place et sa tension.

Ces boucles de maintien se situent de préférence sur deux faces latérales opposées de la guêtre, c'est-à-dire de la jambe dans la position normale d'utilisation du dispositif, ou sur une face arrière de la guêtre, située sur le mollet dans la position normale d'utilisation. Elles sont de préférence portées par deux pattes fixées sur une face arrière de la guêtre, c'est-à-dire positionnées à l'arrière de la jambe dans la position normale d'utilisation, de préférence en une même zone centrale de cette face arrière. Dans des modes de réalisation préférés de l'invention, les pattes sont fixées sur la guêtre de façon à être centrées à l'arrière du mollet.

Une telle configuration se montre particulièrement avantageuse, en ce qu'elle permet d'une part, en combinaison avec le fait que la sangle s'accroche sur l'avant de la jambe, une meilleure répartition des efforts exercés par la sangle en traction sur la jambe, non seulement sur les faces latérales et la face avant de cette dernière, mais aussi sur sa face arrière, si bien que les efforts sont répartis sur toute la surface périphérique de la jambe. In en résulte un meilleur confort d'utilisation du dispositif.

D'autre part, elle permet un meilleur maintien latéral du pied. En effet, dans cette configuration, la sangle vient se superposer sur elle-même au niveau de son point de fixation sur la chaussure, puis ses deux moitiés opposées s'éloignent l'une de l'autre, symétriquement par rapport à l'axe du pied, jusqu'aux deux faces latérales de la jambe, avant de revenir sur elles-mêmes à cet endroit pour rejoindre l'avant de la jambe, ceci toujours symétriquement. Cette trajectoire a pour effet de stabiliser le pied latéralement de façon optimale.

La sangle comporte en outre de préférence, dans sa partie destinée à se replier sur elle-même au niveau du point de fixation sur la chaussure, de préférence dans sa partie centrale, une pièce de renfort destinée à retarder l'usure de la sangle à cet endroit où elle est soumise à d'importants efforts lors de sa traction.

Suivant une caractéristique avantageuse de l'invention, les boucles de maintien sont en outre inclinées de 30 à 60 degrés, et de préférence à environ 45 degrés, vers l'axe de la chaussure. Les pattes qui les supportent présentent de préférence avantageusement la même inclinaison. Ces caractéristiques permettent avantageusement de réaliser un objectif de l'invention qui est de fournir un dispositif de maintien en flexion du pied le plus confortable d'utilisation possible. En effet, une telle inclinaison des boucles et des pattes permet de transmettre avec le moins de résistance l'effort de traction exercé par la sangle sur la guêtre depuis les boucles de maintien vers toute la face avant de la jambe.

Suivant une caractéristique avantageuse de l'invention, la guêtre est constituée en des matériaux semi-flexibles, qui lui donnent une bonne résistance aux efforts exercés sur elle, tout en lui assurant suffisamment de souplesse pour garantir un bon confort d'utilisation.

Dans des modes de réalisation préférés de l'invention, la guêtre est majoritairement constituée en un matériau à propriétés élastiques. Elle consiste de préférence en une bande qui s'attache enroulée autour de la jambe au moyen d'un système de fixation à position ajustable. Ceci permet avantageusement de pouvoir adapter la bande à différents utilisateurs de morphologies différentes, mais aussi, pour un même utilisateur, de pouvoir fixer la bande avec différents degrés de serrage autour de la jambe, suivant que cette dernière est plus ou moins enflée en fonction du degré de fatigue de l'utilisateur.

Le système de fixation ajustable consiste de préférence en des pattes auto-accrochantes, du type à boucles et crochets coopérants, dont l'une est fixée entièrement par-dessus la bande, à une extrémité de celle-ci, et l'autre est fixée à l'autre extrémité de la bande, mais en prolongement de cette dernière. On évite ainsi tout à fait avantageusement la superposition d'un trop grand nombre de couches de matière lorsque la bande est attachée enroulée autour de la jambe pour y former une guêtre. Ceci améliore encore le confort d'utilisation du dispositif selon l'invention.

De plus, dans le même objectif d'assurer une bonne tenue de la guêtre sur la jambe, dans des modes de réalisation préférés de l'invention, la guêtre est équipée de moyens de rigidification qui agissent sur la guêtre en raidissement parallèlement à l'axe de la jambe, et de préférence par un effet de raideur élastique. De tels moyens sont notamment constitués d'au moins deux, et de préférence quatre, baguettes se disposant parallèlement à l'axe de la jambe. Dans le cas particulièrement préféré de l'utilisation de quatre baguettes, ces dernières sont disposées de façon à encadrer deux par deux chacune des boucles de maintien de la sangle. Ces baguettes permettent avantageusement de rigidifier la guêtre, et donc d'une part de lui conférer une meilleure résistance aux efforts exercés sur elle, tant par la sangle que par les mouvements du mollet de l'utilisateur, et d'autre part d'éviter la formation de plis qui pourraient irriter la jambe, tout en lui conservant malgré tout la souplesse qui lui est nécessaire pour un bon confort d'utilisation. Les moyens de rigidification peuvent également consister en des ressorts à spirales, ou en tout autre moyen de rigidification connu.

Suivant une caractéristique avantageuse de l'invention, la sangle est elle-même constituée en un matériau à propriétés élastiques, si bien qu'elle permet une certaine souplesse dans les mouvements de la marche et que la démarche de l'utilisateur est souple et dynamique. En effet, cette élasticité permet un mouvement relatif au niveau de l'articulation entre la jambe et le pied, et elle amortit les chocs subis par le pied à son entrée en contact avec le sol.

De plus, la sangle est de préférence constituée d'une seule pièce. Cependant, on peut également envisager dans le cadre de l'invention d'utiliser une sangle formée de deux pièces sensiblement identiques et distinctes l'une de l'autre. Ces deux pièces seraient reliées l'une à l'autre à une extrémité au niveau du point de fixation situé sur l'avant de la chaussure, ou bien reliées chacune à ce point de fixation. Elles se rejoindraient à l'autre extrémité sur la face avant de la jambe, le résultat étant semblable à celui obtenu en utilisant une bande formée d'une seule pièce.

Dans des modes de réalisation préférés de l'invention, la sangle est enfilée, de manière à y coulisser librement, dans un passant équipé de crochets qui sont de préférence à libre coulissement et pivotement. Ceci permet son accrochage au lacet de la chaussure, ou à des orifices prévus à cet effet sur une face supérieure avant de cette dernière. Ce système permet avantageusement de pouvoir accrocher la sangle suivant l'invention facilement à tout type de chaussure, en ne nécessitant d'adaptation de cette dernière que dans le cas où elle ne porte pas de lacet. De plus, il est également facile de la décrocher.

La sangle peut également être enfilée dans une boucle formée par le lacet de la chaussure.

Tout autre mode de fixation de la sangle à l'avant de la chaussure à la portée de l'homme du métier entre également dans le cadre de l'invention.

Suivant une caractéristique avantageuse de l'invention, le dispositif est associé à un ruban qui se fixe sur lui-même en position enroulée directement autour du pied non chaussé, et qui comporte une boucle pour l'accrochage au pied de la sangle, ce qui permet d'utiliser le dispositif selon l'invention en l'absence de chaussure.

L'invention sera maintenant plus complètement décrite dans le cadre de caractéristiques préférées et de leurs avantages, en faisant référence aux figures 1 à 4 dans lesquelles :
- la figure 1 représente une sangle d'un dispositif suivant l'invention ;
- la figure 2 montre une vue en plan d'une bande destinée à former guêtre enroulée autour de la jambe, en conformation déroulée ;
- la figure 3 illustre une étape du procédé de mise en place du dispositif suivant l'invention sur la jambe d'une personne ;
- et la figure 4 représente le ruban à installer directement autour du pied.

Le dispositif suivant l'invention est entièrement constitué de matériaux qui sont légers et qui n'engendrent aucun risque de blessure pour l'utilisateur.

Il se compose d'une guêtre destinée à être portée autour de la partie inférieure de la jambe et d'une sangle destinée à être tendue entre un point situé à l'avant de la chaussure et deux points latéraux de la guêtre opposés de part et d'autre de l'axe de la chaussure.

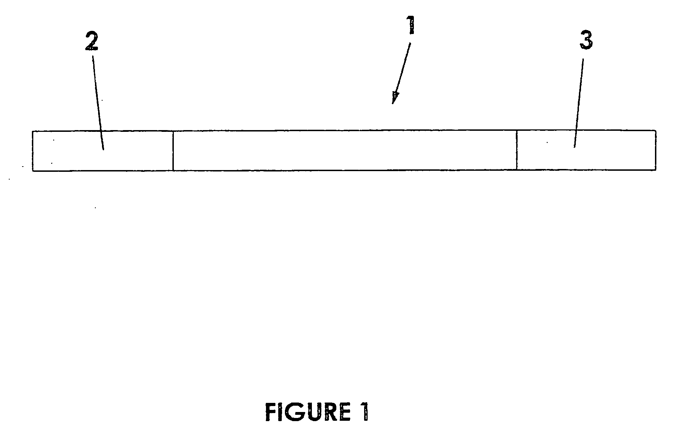

La sangle 1 est représentée sur la figure 1. Elle est constituée d'une bande en un matériau à propriétés élastiques, notamment à base d'une pâte gommée tissée. Elle présente de préférence une capacité d'allongement de 70 à 90 %.

Il est avantageusement prévu dans le cadre de l'invention différents formats de sangle, ainsi que de guêtres, pour s'adapter aux différentes morphologies des utilisateurs. Dans des modes de réalisation préférés de l'invention, la largeur de la sangle est environ comprise entre 2 et 3,5 cm. Sa longueur peut varier entre 40 et 90 cm.

A ses deux extrémités, la sangle 1 est munie de pattes de fixation 2 et 3 qui servent à fermer la sangle sur elle-même à une longueur ajustable. Ces pattes sont destinées à se fixer l'une sur l'autre dans la position finale d'utilisation. Il peut s'agir notamment, dans des modes de réalisation préférés de l'invention, de pattes auto-accrochantes, du type à boucles et crochets. Les deux pattes de fixation 2 et 3 sont disposées sur la même face de la sangle 1. La longueur des pattes de fixation peut varier suivant les cas particuliers d'application de l'invention, notamment entre 7 et 15 cm. Leur largeur est environ égale à la largeur de la sangle 1. Ces dimensions permettent d'obtenir une fixation de la sangle sur elle-même de grande solidité.

La sangle 1 est associée au sein du dispositif suivant l'invention à une bande 4 destinée à être enroulée pour former guêtre autour de la jambe de l'utilisateur. La bande 4 est constituée en un matériau à propriété élastique, qui est à la fois suffisamment souple pour ne pas gêner ou blesser l'utilisateur, et suffisamment rigide pour offrir une bonne résistance mécanique, notamment sous l'effet de la tension exercée par la sangle tendue entre l'avant du pied et la guêtre, ainsi qu'un bon maintien sur la jambe. La bande 4 peut notamment être constituée d'un mélange de coton et de fibres élastiques.

Selon des modes de réalisation préférés de l'invention, la bande 4 présente une hauteur comprise entre environ 10 et 20 cm. Sa hauteur est notamment environ égale à 14 à 16 cm. Cependant ces dimensions ne sont en aucun cas restrictives de l'invention, et tout autre format entre également dans le cadre de l'invention, dans la mesure où il n'entraîne pas une entrave des mouvements de l'utilisateur. En particulier, on peut imaginer suivant l'invention différents formats de bandes qui seraient adaptés à différents types d'utilisateurs, en distinguant notamment hommes, femmes ou enfants.

La bande 4 est associée à ses deux extrémités à des pattes de fixation 5 et 6 destinées à se fixer l'une sur l'autre lorsque la bande est enroulée autour de la jambe, afin de la maintenir bloquée dans cette position. Ces pattes de fixation 5 et 6 sont de préférence auto-accrochantes. Il s'agit notamment de pattes du type à boucles et crochets qui se fixent l'une sur l'autre en une position réglable, ce qui permet d'ajuster la bande autour de jambes de diamètres différents.

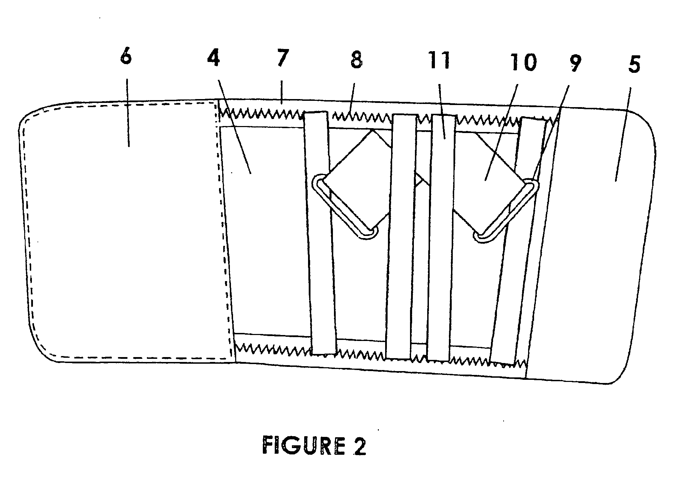

La première patte de fixation 5 est fixée, notamment cousue, sur la bande 4, de manière à en occuper sensiblement toute une partie terminale. La seconde patte de fixation 6 est fixée à l'extrémité opposée de la bande 4, en prolongement de cette dernière, et sans se superposer à elle. Les pattes de fixation 5 et 6 sont orientées dans le sens de la hauteur et apposées respectivement sur deux faces opposées de la bande 4, si bien qu'elles se trouvent en regard l'une de l'autre lorsque la bande est enroulée autour de la jambe. Dans le mode de réalisation préféré illustré en plan sur la figure 2, la patte de fixation 5 est disposée sur la face de la bande 4 visible sur la figure, alors que la patte de fixation 6 se trouve sur son autre face, représentée en pointillé sur la figure.

Les pattes de fixation 5 et 6 occupent de préférence toute la hauteur de la bande 4. Leur largeur peut prendre diverses valeurs. Elle peut être notamment comprise entre 1 et 8 cm pour la patte 5 cousue sur la bande 4, et entre 2 et 15 cm pour la patte 6 cousue à l'extrémité de cette dernière. On obtient ainsi avantageusement une fixation solide de la bande 4 autour de la jambe. La patte de fixation 6, qui est notamment la patte à surface à boucles, présente de préférence une largeur plus importante. In est de ce fait possible de positionner la patte coopérante 5 en différentes positions à sa surface. Cette caractéristique permet un réglage personnalisé du degré de serrage de la guêtre autour de la jambe, en fonction de l'utilisateur particulier.

Sur ses deux bords longitudinaux, la bande 4 est renforcée par une pièce 7 de forme étroite et allongée en une matière élastique. Sa hauteur est environ égale à 3 cm. Elle est fixée sur la bande 4 au moyen d'une ligne de couture en zigzag 8, qui donne une meilleure solidité à la fixation. Ceci permet avantageusement d'une part de renforcer la bande 4 le long de ses deux bords longitudinaux, et ce sur toute sa longueur, et d'autre part d'en conserver tout de même la flexibilité.

Sur la bande 4 sont fixées deux boucles 9, par l'intermédiaire de pattes 10 souples mais non élastiques. Ces boucles sont destinées à maintenir, au niveau de la guêtre, la sangle 1, qui sera enfilée et qui pourra coulisser librement à travers elles. Les boucles 9 se présentent de préférence sous la forme d'anneaux de section bombée ou plate, constitués en un matériau à la fois léger et résistant, notamment à base de nickel ou de plastique.

Les pattes 10 sont fixées sur la partie supérieure de la bande 4, c'est-à-dire celle qui est destinée à s'appliquer sur la partie haute de la jambe, vers le genou. Elles sont dimensionnées et positionnées de telle sorte que les boucles 9 se situent également dans la partie supérieure de la bande 4. Dans le sens longitudinal, elles sont positionnées au voisinage de la partie médiane de la bande. De la sorte, lorsque la bande 4 est enroulée autour de la jambe et fermée sur le devant du tibia, les pattes 10 se disposent à l'arrière de la jambe, environ centrées sur le mollet, et chacune des boucles 9 se situe sur une face latérale de la jambe, très légèrement vers l'arrière. En pratique, pour obtenir cette disposition, les pattes 10 sont positionnées et dimensionnées de telle sorte que les boucles 9 sont espacées l'une de l'autre, à leurs extrémités, d'une distance environ égale à 4 à 5 cm, bien que cette distance puisse varier dans le cadre de l'invention, en fonction du format particulier de la bande destinée à un type d'utilisateur donné.

Les boucles 9 sont inclinées par rapport à un axe transversal de la bande 4. L'inclinaison des boucles par rapport à cet axe est notamment égale à environ 45 degrés. Ainsi, dans la position normale d'utilisation de la bande, c'est-à-dire la bande enroulée autour de la jambe, les boucles sont inclinées d'environ 45 degrés par rapport à l'axe de la jambe, ce qui donne une meilleur ergonomie au dispositif.

Les pattes 10 sont également inclinées par rapport à un axe transversal de la bande 4, les boucles 9 leur étant généralement perpendiculaires. Ainsi, dans le mode de réalisation préféré représenté sur la figure, elles sont jointives à leur bout fixe dans la partie supérieure de la bande 4, et elles s'éloignent l'une de l'autre en allant vers le bas, jusqu'à leur bout libre portant les boucles 9.

La bande 4 est munie de quatre gaines 11, qui lui sont transversales, et qui sont disposées de manière à encadrer deux par deux chaque boucle 9. Ainsi, sont disposées sur la bande 4 deux gaines 11 situées entre les deux boucles 9, et deux gaines 11 situées de part et d'autre de ces boucles. Ces gaines transversales occupent sensiblement toute la hauteur de la bande 4. Elles sont de préférence constituées en cuir ou en un sergé croisé de coton, ce qui leur confère de la solidité. Elles sont destinées à recevoir des moyens de raidissement de la bande 4 suivant son axe transversal.

De tels moyens de raidissement sont constitués, dans des modes de réalisation préférés de l'invention, par des baguettes. Ces baguettes permettent, une fois enfilées dans les gaines correspondantes 11, de rigidifier la bande 4. Par conséquent, elles lui confèrent une meilleure résistance à la tension exercée sur elle par la sangle 1 enfilée dans les boucles 9. Il en résulte aussi une meilleure tenue autour de la jambe lorsque cette dernière est en mouvement. De plus, les baguettes permettent également d'éviter la formation de plis sur la bande 4, plis qui pourraient occasionner une gêne, voire des blessures, pour l'utilisateur. Les baguettes sont plus courtes que les gaines 11 dans la partie inférieure de la bande, si bien qu'elles ne risquent pas de blesser la cheville au niveau de la malléole, lorsque la bande est en position opérationnelle autour de la jambe.

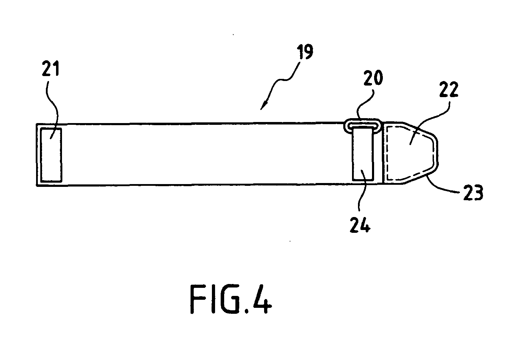

Le dispositif est en outre associé à un ruban 19, qui est illustré sur la figure 4, et qui est destiné à être enroulé autour du pied, vers l'extrémité avant de ce dernier, lorsque l'utilisateur ne souhaite pas utiliser de chaussure.

Ce ruban 19 est constitué en un matériau souple à propriétés élastiques. Il présente un largeur d'environ 3 à 7 cm, de sorte à être confortable d'utilisation, et une longueur de l'ordre de 20 à 40 cm, de sorte à s'adapter à différentes morphologies.

Il se ferme sur lui-même, enroulé autour du pied, au moyen de pattes de fixation 21 et 22 fixées à ses extrémités opposées, respectivement sur ses faces opposées. Ces pattes 21 et 22 sont de préférence des pattes auto-accrochantes, notamment du type à boucles et crochets. Dans le mode de réalisation préféré illustré en plan sur la figure 4, la patte de fixation 21 est disposée sur la face du ruban visible sur la figure, alors que la patte de fixation 22 se trouve sur son autre face, représentée en pointillé sur la figure.

A une de ses extrémités 23, le ruban 19 présente une forme effilée qui facilite sa préhension pour désolidariser les pattes 21 et 22 fixées l'une à l'autre.

Sur une de ses faces, le ruban 19 porte une patte 24 qui maintient une boucle 20. Le ruban 19 est destiné à être installé sur le pied de telle sorte que cette boucle 20 soit disposée sur le haut du pied, faisant face à la jambe. Elle peut alors recevoir la sangle 1 pour l'accrochage de cette dernière à l'avant du pied.

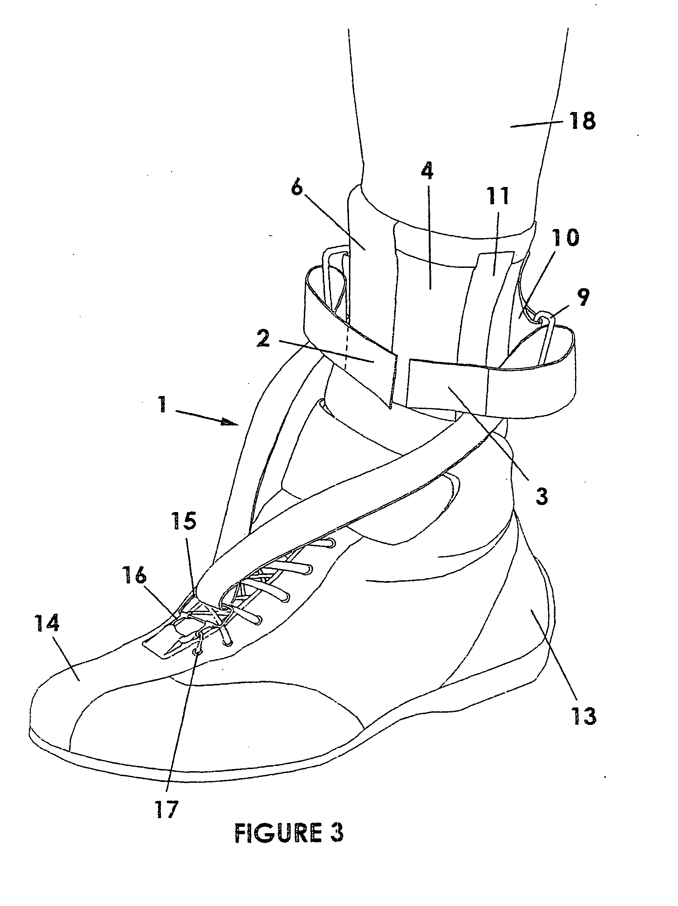

La mise en place du dispositif suivant l'invention sera maintenant décrite plus en détail en se basant sur la figure 3.

La bande 4 est enroulée autour de la jambe 18, sur la partie inférieure de celle-ci, de préférence juste au-dessus de la malléole. De cette façon, la bande ne gêne pas le mouvement de la cheville, mais elle permet un bon maintien de cette dernière en combinaison avec la sangle. La bande 4 enroulée autour de la jambe est fixée dans cette position par accrochage des pattes de fixation 5 et 6 l'une sur l'autre sur le devant de la jambe. Cette fixation peut être effectuée dans différentes positions, en fonction de la taille de la jambe de l'utilisateur. La bande 4 doit être suffisamment serrée autour de la jambe pour bien tenir en place lors des différents mouvements que peut effectuer l'utilisateur, mais pas trop cependant pour ne pas gêner ou blesser l'utilisateur, et en particulier ne pas perturber la circulation sanguine.

La bande 4 est positionnée autour de la jambe de telle sorte que les pattes 10 soient centrées sur le mollet, et que chacune des boucles 9 se situe sur une face latérale opposée, légèrement vers l'arrière, de la jambe. Les boucles 9 étant fixées sur la bande 4 par l'intermédiaire des pattes 10, elles peuvent être décollées de la surface de la bande, ce qui facilite l'insertion de la sangle 1 à travers elles.

Dans cette disposition, les gaines 11 de réception des baguettes sont situées, pour deux d'entre elles sur les faces latérales de la jambe, et, pour les deux autres, à l'arrière de la jambe, de chaque côté du mollet. Ainsi, l'ensemble est à la fois rigide et souple, si bien qu'il présente une bonne tenue aux efforts qui peuvent être exercés sur lui, mais qu'il assure cependant un bon confort d'utilisation à la personne.

Une fois la bande 4 mise en place autour de la jambe 18 de façon à former une guêtre, une chaussure 13 ou autre chaussant, tel que le ruban 19 illustré sur la figure 4, est installée sur le pied. La chaussure 13 est en elle-même classique. Cependant, il est tout à fait avantageux dans le cadre de l'invention d'utiliser une chaussure montante, qui tiendra mieux la cheville et qui viendra se positionner par son extrémité supérieure au-dessus de la bordure inférieure de la bande 4. Ainsi, la chaussure 13 participera à un meilleur maintien en place de la bande 4. La chaussure 13 est de préférence munie d'un lacet 17. Dans le cas contraire, il est nécessaire de l'adapter pour permettre la fixation de la sangle 1 sur sa face avant supérieure, par exemple par perçage d'un ou deux orifices sur cette face et mise en place d'un lien entre ces orifices.

La sangle 1 est ensuite mise en place, de manière à ce qu'elle s'étende depuis une face supérieure avant 14 de la chaussure 13, par les deux boucles 9 situées de part et d'autre de la jambe, jusqu'à un point de fixation situé sur la face avant de la jambe 18, comme représenté sur la figure 3.

Pour cela, la sangle 1 est fixée à l'avant de la chaussure 13 sensiblement en son milieu. Elle peut être fixée par différents moyens. Dans le mode de réalisation préféré du dispositif selon l'invention tel que représenté sur la figure 3, la sangle 1 est associée à un passant 15 dans lequel elle est enfilée et à travers lequel elle coulisse librement. Le passant 15 est constitué en un matériau rigide à la fois léger et résistant. Il est notamment constitué en un métal à base de nickel ou en plastique. Sur le passant 15 sont fixés, de manière à pouvoir coulisser et pivoter librement, deux crochets 16 qui sont constitués également en un matériau rigide et léger. Ces deux crochets permettent avantageusement de fixer le passant 15, dans lequel la sangle 1 est enfilée, sur le lacet 17 porté par la chaussure 13 représentée sur la figure 3. Dans le cas d'une chaussure qui ne comporterait pas de lacet, ces crochets 16 pourraient venir s'accrocher soit dans des orifices qui seraient ménagés à cet effet sur la face supérieure avant 14 de la chaussure, soit sur un lien reliant de tels orifices.

Le mode de réalisation qui vient d'être décrit n'est nullement limitatif de l'invention. En effet, on peut également envisager dans le cadre de l'invention de fixer la sangle 1 sur la chaussure par différents autres moyens. Par exemple, la sangle 1 peut être directement enfilée dans une boucle formée par le lacet 17 de la chaussure. Suivant un autre mode de réalisation avantageux de l'invention, un ou deux orifices peuvent être percés sur la sangle 1, dans sa partie centrale, de sorte que le lacet 17 puisse être enfilé à travers ce ou ces orifices. Ceci permettrait également de fixer efficacement la sangle au lacet. On peut également envisager d'associer à ce ou ces orifices un crochet destiné à s'accrocher sur le lacet 17.

Depuis son point de fixation à l'avant de la chaussure, la sangle 1 s'étend symétriquement, en deux branches formant l'une avec l'autre un angle d'environ 25 degrés, vers l'arrière de la jambe, jusqu'aux deux boucles 9, à travers lesquelles elle est insérée respectivement par chacune de ses extrémités. Dans cette configuration, la sangle 1 se superpose sur elle-même sur la partie avant de la chaussure, près de son point de fixation. Elle s'étend ensuite de part et d'autre du pied jusqu'aux boucles 9. Depuis les boucles 9, elle est ramenée par ses deux extrémités vers l'avant de la jambe. Elle est fixée sur le devant de la jambe par fermeture sur elle-même au moyen des deux pattes de fixation 2 et 3. Cette opération est réalisée en tendant la sangle 1 suffisamment pour que l'avant du pied soit ramené vers la face avant de la jambe, de telle sorte que le pied forme sensiblement un angle droit avec la jambe.

Le pied est alors maintenu au moyen de ce dispositif en position fléchie par rapport à la jambe. Pour marcher, l'utilisateur atteint du syndrome du pied équin n'a alors plus besoin de lancer exagérément la jambe vers l'avant. Il peut poser le talon sur le sol de façon normale, puis abaisser l'avant-pied sous l'effet d'une simple poussée exercée par la cuisse avant de relever à nouveau la jambe. Cette opération n'engendre aucune douleur pour l'utilisateur, puisque la tension exercée par la sangle est uniformément répartie sur toute la périphérie de la jambe. Le dispositif suivant l'invention est en outre confortable à porter. De plus, il présente l'avantage de laisser travailler les muscles de la jambe de l'utilisateur, si bien qu'il permet une rééducation active par les mouvements mêmes de la marche. Enfin, le pied est bien maintenu latéralement.

La description qui précède explique clairement comment l'invention permet d'atteindre les objectifs qu'elle s'est fixés. En particulier, elle fournit un dispositif de maintien du pied en position fléchie par rapport à la jambe, qui est léger, facile à mettre en place et confortable à porter, qui assure un bon maintien latéral de la cheville et qui n'engendre aucune douleur particulière au niveau de la jambe pour l'utilisateur. Le dispositif suivant l'invention permet aux personnes atteintes d'un déficit fonctionnel du pied de marcher de façon plus naturelle, dynamique et souple que les dispositifs existants. De plus, il ne donne pas à l'utilisateur l'impression d'être appareillé et il réduit la transpiration. Il apporte à l'utilisateur un bien-être physique et psychologique que ne permettent pas les autres dispositifs existants.

Il ressort néanmoins de ce qui précède que l'invention n'est pas limitée aux modes de mise en oeuvre qui ont été spécifiquement décrits et représentés sur les figures, et qu'elle s'étend au contraire à toutes variantes passant par le biais de moyens équivalents.

## Revendications

1. Dispositif de maintien en flexion du pied d'une personne, comportant des moyens tenseurs pour relever l'extrémité avant du pied vers une guêtre (4) se disposant autour de la jambe correspondante, **caractérisé en ce que** lesdits moyens tenseurs sont constitués par une sangle (1) en matériau élastique à capacité d'allongement de 70 à 90%, **en ce qu'**il comporte un passant (15) équipé de crochets (16) pour l'accrochage de ladite sangle à un lacet (17) d'une chaussure (13), **en ce que** la guêtre a deux boucles de maintien (9) fixées sur elle en deux points latéraux se disposant sur des zones latérales opposées de la jambe, de part et d'autre de l'axe du pied, et **en ce que** la sangle se dispose en deux branches s'étendant symétriquement à partir d'un point de fixation à l'avant de la chaussure vers deux points latéraux de ladite guêtre se disposant sur des zones latérales opposées de la jambe, de part et d'autre de l'axe du pied, en passant chacune par une des boucles de maintien (9) dans laquelle elle coulisse librement.

2. Dispositif de maintien en flexion du pied d'une personne, comportant des moyens tenseurs pour relever l'extrémité avant du pied vers une guêtre (4) se disposant autour de la jambe correspondante, **caractérisé en ce que** lesdits moyens tenseurs sont constitués par une sangle (1) en matériau élastique à capacité d'allongement de 70 à 90%, **en ce qu'**il comporte un ruban (19) adapté à se fixer sur lui-même en position enroulée autour de l'avant du pied, le ruban comportant une boucle (20) pour l'accrochage au pied de ladite sangle, **en ce que** la guêtre a deux boucles de maintien (9) fixées sur elle en deux points latéraux se disposant sur des zones latérales opposées de la jambe, de part et d'autre de l'axe du pied, et **en ce que** la sangle se dispose en deux branches s'étendant symétriquement à partir de la boucle (20) du ruban, en passant chacune par une des boucles de maintien (9) dans laquelle elle coulisse librement.

3. Dispositif selon la revendication 1, **caractérisé en ce que** ladite sangle (1) s'enfile dans ledit passant (15), qui est équipé de crochets (16) venant s'accrocher sur un lacet de chaussure (17) ou dans des orifices prévus à cet effet sur la face supérieure avant (14) d'une chaussure, ou sur un lien entre de tels orifices,

4. Dispositif selon l'une des revendications 1 à 3, **caractérisé en ce que** ladite guêtre consiste en une bande (4) qui s'attache enroulée autour de la jambe au moyen d'un système de fixation à position ajustable (5, 6) et **en ce que** lesdites boucles (9) de maintien de ladite sangle (1), qui se situent sur deux faces latérales opposées de ladite guêtre ou sur une face arrière de cette dernière, sont portées par deux pattes (10) fixées sur une face arrière de ladite guêtre, de préférence en une même zone centrale de ladite face arrière.

5. Dispositif selon la revendication 4, **caractérisé en ce que** les pattes de fixation des boucles (9) de maintien de la sangle sont inclinées de 30 à 60 degrés vers l'axe du pied, de préférence à environ 45 degrés.

6. Dispositif selon l'une des revendications 1 à 5, **caractérisé en ce que** ladite guêtre est équipée de moyens de rigidification agissant sur la guêtre en raidissement parallèlement à l'axe de la jambe.

7. Dispositif suivant la revendication 6, dans lequel lesdits moyens de rigidification sont constitués d'au moins deux baguettes se disposant parallèlement à l'axe de la jambe.

8. Dispositif selon l'une quelconque des revendications précédentes, **caractérisé en ce que** lesdits deux points de ladite guêtre sont espacés l'un de l'autre de telle sorte que ladite sangle (1) forme avec elle-même, entre ledit point de fixation à l'avant du pied et lesdits deux points de ladite guêtre, un angle compris entre 10 et 40 degrés, de préférence environ égal à 25 degrés.

9. Dispositif selon l'une des revendications précédentes, **caractérisé en ce que** ladite sangle (1) s'accroche par ses deux extrémités sur la face avant de la jambe, en blocage à une longueur assurant un effet tenseur approprié pour relever l'extrémité avant du pied vers la jambe portant ladite guêtre.

10. Dispositif selon la revendication 9, **caractérisé en ce que** ladite sangle (1) se ferme en boucle sur elle-même sur la face avant de la jambe, notamment au moyen d'un système de fixation à position ajustable (2, 3).

11. Dispositif selon l'une quelconque des revendications précédentes, **caractérisé en ce que** ladite sangle se fixe à l'avant du pied sensiblement en son milieu.

## Patentansprüche

1. Vorrichtung zum Halten des Fußes einer Person in einer gebeugten Stellung, wobei die Vorrichtung Spannmittel zum Anheben des vorderen Endes des Fußes zu einer Gamasche (4) hin aufweist, die um das entsprechende Bein herum angeordnet wird, **dadurch gekennzeichnet, dass** die Spannmittel aus einem Gurt (1) aus elastischem Material mit einer Dehnfähigkeit von 70 bis 90 % bestehen, dass die Vorrichtung eine Schlaufe (15) aufweist, die mit Haken (16) zum Einhängen des Gurts in einen Schnürsenkel (17) eines Schuhs (13) ausgestattet ist, dass die Gamasche zwei Halteringe (9) besitzt, die an ihr an zwei seitlichen Punkten befestigt sind, welche an entgegengesetzten seitlichen Bereichen des Beins zu beiden Seiten der Achse des Fußes angeordnet sind, und dass der Gurt in zwei Strängen angeordnet wird, die sich symmetrisch von einem Befestigungspunkt an der Vorderseite des Schuhs aus zu zwei seitlichen Punkten der Gamasche erstrecken, die an entgegengesetzten seitlichen Bereichen des Beins zu beiden Seiten der Achse des Fußes angeordnet sind, wobei jeder durch einen der Halteringe (9) verläuft, in dem er frei gleiten kann.

2. Vorrichtung zum Halten des Fußes einer Person in einer gebeugten Stellung, wobei die Vorrichtung Spannmittel zum Anheben des vorderen Endes des Fußes zu einer Gamasche (4) hin aufweist, die um das entsprechende Bein herum angeordnet wird, **dadurch gekennzeichnet, dass** die Spannmittel aus einem Gurt (1) aus elastischem Material mit einer Dehnfähigkeit von 70 bis 90 % bestehen, dass die Vorrichtung ein Band (19) umfasst, das geeignet ist, in der um die Spitze des Fußes gewickelten Position auf sich selbst befestigt zu werden, wobei das Band einen Ring (20) zum Anhängen des Gurts an den Fuß aufweist, dass die Gamasche zwei Halteringe (9) besitzt, die an ihr an zwei seitlichen Punkten befestigt sind, welche an entgegengesetzten seitlichen Bereichen des Beins zu beiden Seiten der Achse des Fußes angeordnet sind, und dass der Gurt in zwei Strängen angeordnet wird, die sich symmetrisch vom Ring (20) des Bandes aus erstrecken, wobei jeder durch einen der Halteringe (9) verläuft, in dem er frei gleiten kann.

3. Vorrichtung nach Anspruch 1, **dadurch gekennzeichnet, dass** der Gurt (1) durch die Schlaufe (15) gezogen wird, die mit Haken (16) ausgestattet ist, die in einen Schnürsenkel (17) oder in Öffnungen, die zu diesem Zweck an der vorderen Oberseite (14) eines Schuhs vorgesehen sind, oder in eine Verbindung zwischen diesen Öffnungen eingehängt wird.

4. Vorrichtung nach einem der Ansprüche 1 bis 3, **dadurch gekennzeichnet, dass** die Gamasche aus einem Streifen (4) besteht, der um das Bein herum gewickelt mittels eines verstellbaren Befestigungssystems (5, 6) befestigt wird, und dass die Halteringe (9) des Gurts (1), die sich an zwei entgegengesetzten seitlichen Flächen der Gamasche oder an einer Rückseite der Gamasche befinden, von zwei Laschen (10) getragen werden, die an einer Rückseite der Gamasche befestigt sind, vorzugsweise in demselben mittleren Bereich der Rückseite.

5. Vorrichtung nach Anspruch 4, **dadurch gekennzeichnet, dass** die Befestigungslaschen der Halteringe (9) des Gurts um 30 bis 50 Grad, vorzugsweise um etwa 45 Grad, zur Achse des Fußes hin geneigt sind.

6. Vorrichtung nach einem der Ansprüche 1 bis 5, **dadurch gekennzeichnet, dass** die Gamasche mit Aussteifungsmitteln ausgestattet ist, die auf die Gamasche parallel zur Achse des Beins versteifend wirken.

7. Vorrichtung nach Anspruch 6, bei der die Aussteifungsmittel aus mindestens zwei Stäben bestehen, die parallel zur Achse des Beins angeordnet sind.

8. Vorrichtung nach einem der vorangehenden Ansprüche, **dadurch gekennzeichnet, dass** die beiden Punkte der Gamasche so voneinander beabstandet sind, dass der Gurt (1) mit sich selbst zwischen dem Befestigungspunkt an der Spitze des Fußes und den beiden Punkten der Gamasche einen Winkel von 10 bis 40 Grad vorzugsweise von etwa 25 Grad, bildet.

9. Vorrichtung nach einem der vorangehenden Ansprüche, **dadurch gekennzeichnet, dass** der Gurt (1) an seinen beiden Enden an der Vorderseite des Beins angehängt wird und auf einer Länge festgestellt wird, die eine geeigneten Spannwirkung gewährleistet, um das vordere Ende des Fußes zu dem die Gamasche tragenden Bein anzuheben.

10. Vorrichtung nach Anspruch 9, **dadurch gekennzeichnet, dass** sich der Gurt (1) als Schlinge auf sich selbst an der Vorderseite des Beins schließt, insbesondere mit einem Befestigungssystem mit verstellbarer Position (2, 3).

11. Vorrichtung nach einem der vorangehenden Ansprüche, **dadurch gekennzeichnet, dass** der Gurt an der Spitze des Fußes im Wesentlichen in dessen Mitte befestigt wird.

## Claims

1. Device for retaining a person's foot in a flexed position, comprising tensor means to raise the front end of the foot towards a gaiter (4) which is arranged around the corresponding leg, **characterised in that** the said tensor means are constituted by a strap (1) made of resilient material with an elongation capacity of 70 to 90%, **in that** it comprises a keeper (15) equipped with hooks (16) for hooking of the said strap on a lace (17) of a shoe (13), **in that** the gaiter has two retention loops (9) which are secured on it at two lateral points arranged on opposite lateral areas of the leg, on both sides of the axis of the foot, and **in that** the strap is arranged in two branches which extend symmetrically from a securing point at the front of the shoe, towards two lateral points of the said gaiter which are arranged on opposite lateral areas of the leg, on both sides of the axis of the foot, whilst each passing via one of the retention loops (9) in which it slides freely.

2. Device for retaining a person's foot in a flexed position, comprising tensor means to raise the front end of the foot towards a gaiter (4) which is arranged around the corresponding leg, **characterised in that** the said tensor means are constituted by a strap (1) made of resilient material with an elongation capacity of 70 to 90%, **in that** it comprises a tape (19) which is designed to be secured to itself in the position in which it is wound around the front of the foot, the tape comprising a loop (20) for the hooking of the said strap on the foot, **in that** the gaiter has two retention loops (9) which are secured on it at two lateral points arranged on opposite lateral areas of the leg, on both sides of the axis of the foot, and **in that** the strap is arranged in two branches which extend symmetrically from the loop (20) of the tape, whilst each passing via one of the retention loops (9) in which it slides freely.

3. Device according to claim 1, **characterised in that** the said strap (1) is threaded into the said keeper (15) which is equipped with hooks (16) which are hooked onto a lace (17) of a shoe or into orifices provided for this purpose on the front upper surface (14) of a shoe, or onto a link between orifices of this type.

4. Device according to one of claims 1 to 3, **characterised in that** the said gaiter consists of a band (4) which is attached by being wound around the leg by means of a securing system with an adjustable position (5, 6), and **in that** the said loops (9) for retention of the said strap (1), which are situated on two opposite lateral surfaces of the said gaiter, or on a rear surface of the latter, are supported by two lugs (10) which are secured on a rear surface of the said gaiter, and preferably in the same central area of the said rear surface.

5. Device according to claim 4, **characterised in that** the lugs for securing of the loops (9) for retention of the strap are inclined by 30 to 60 degrees towards the axis of the foot, and preferably by approximately 45 degrees.

6. Device according to one of claims 1 to 5, **characterised in that** the said gaiter is equipped with stiffening means which act on the gaiter by stiffening parallel to the axis of the leg.

7. Device according to claim 6, wherein the said stiffening means are constituted by at least two strips which are arranged parallel to the axis of the leg.

8. Device according to any one of the preceding claims, **characterised in that** the said two points of the said gaiter are spaced from one another such that the said strap (1) forms with itself, between the said securing point at the front of the foot and the said two points of the said gaiter, an angle contained between 10 and 40 degrees, and preferably approximately equal to 25 degrees.

9. Device according to one of the preceding claims, **characterised in that** the said strap (1) is hooked by its two ends on the front surface of the leg, with blocking at a length which provides a tensor effect which is appropriate for raising the front end of the foot towards the leg which is wearing the said gaiter.

10. Device according to claim 9, **characterised in that** the said strap (1) is closed in a loop on itself on the front surface of the leg, in particular by means of a securing system with an adjustable position (2, 3).

11. Device according to any one of the preceding claims, **characterised in that** the said strap is secured at the front of the foot, substantially in its middle.
